(19) Europäisches Patentamt
European Patent Office
Office européen des brevets

(11) **EP 3 073 924 B1**

(12) **EUROPÄISCHE PATENTSCHRIFT**

(45) Veröffentlichungstag und Bekanntmachung des Hinweises auf die Patenterteilung:
**06.10.2021 Patentblatt 2021/40**

(21) Anmeldenummer: **15707934.4**

(22) Anmeldetag: **03.03.2015**

(51) Int Cl.:
**A61B 6/00** (2006.01)   **G06T 7/00** (2017.01)

(86) Internationale Anmeldenummer:
**PCT/EP2015/054429**

(87) Internationale Veröffentlichungsnummer:
**WO 2015/169470 (12.11.2015 Gazette 2015/45)**

(54) **AUSWERTUNG EINES WÄHREND EINER MAMMOGRAPHIE ERZEUGTEN RÖNTGENBILDES EINER BRUST**

EVALUATION OF AN X-RAY IMAGE OF A BREAST PRODUCED DURING A MAMMOGRAPHY

ÉVALUATION D'UNE RADIOGRAPHIE D'UN SEIN GÉNÉRÉE PENDANT UNE MAMMOGRAPHIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.05.2014 DE 102014208411**

(43) Veröffentlichungstag der Anmeldung:
**05.10.2016 Patentblatt 2016/40**

(73) Patentinhaber: **Siemens Healthcare GmbH**
**91052 Erlangen (DE)**

(72) Erfinder: **FIESELMANN, Andreas**
**91052 Erlangen (DE)**

(56) Entgegenhaltungen:
**DE-A1-102006 021 042    US-A1- 2011 026 791**

- **COLIN C ET AL: "Can mammographic assessments lead to consider density as a risk factor for breast cancer?", EUROPEAN JOURNAL OF RADIOLOGY, ELSEVIER SCIENCE, NL, Bd. 82, Nr. 3, 4. Februar 2010 (2010-02-04), Seiten 404-411, XP028978963, ISSN: 0720-048X, DOI: 10.1016/J.EJRAD.2010.01.001**
- **None**

**Beschreibung**

Die Erfindung betrifft ein Verfahren, eine Vorrichtung und ein Computerprogramm zur Auswertung eines während einer Mammographie erzeugten Röntgenbildes einer Brust.

Es ist bekannt, aus einem während einer Mammographie erzeugten Röntgenbild einer Brust die volumetrische Brustdichte (VBD) automatisch zu berechnen. Die volumetrische Brustdichte ist definiert als das Verhältnis des Volumens des fibro-glandulären Gewebes zu dem Gesamtvolumen der Brust. Nachfolgend werden die Begriffe "fibro-granduläres Gewebe", "granduläres Gewebe" und "mammographisch dichtes Gewebe" bzw. "dichtes Gewebe" synonym verwendet. Auf der Grundlage dieses VBD-Wertes wird bisher der Brust unter Verwendung fester Grenzwerte eine bestimmte Brustdichtekategorie zugeordnet, insbesondere ein BI-RADS-Wert "1" bis "4" gemäß der Einteilung des American College of Radiology (ACR). Dies ist bespielsweise in US 2011/0026791 A1 und in DE 10 2006 021 042 A1 beschrieben.

Bei Frauen, deren Brust einen hohen VBD-Wert aufweist, besteht ein erhöhtes Risiko einer Brustkrebserkrankung. Die Erhöhung dieses Risikos wird teilweise auf die Tatsache zurückgeführt, dass kanzeröses Gewebe durch mammographisch dichtes Gewebe maskiert und daher während der Mammographie nicht erkannt wird.

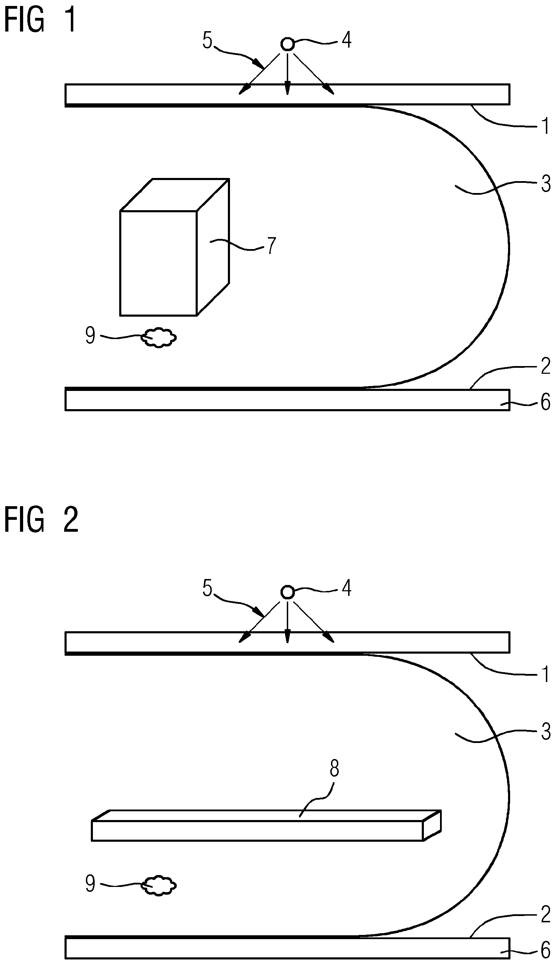

Es ist bekannt, dass das Maskierungsrisiko nicht immer mit dem VBD-Wert korreliert. In den FIG 1 und 2 ist eine zwischen zwei Platten 1, 2 komprimierte Brust 3 während einer Mammographie dargestellt. Von einer Röntgenquelle 4 ausgehende Röntgenstrahlung 5 trifft nach dem Durchtritt durch das Gewebe der Brust 3 auf einen Röntgendetektor 6. Wie in FIG 1 und 2 dargestellt, kann das gleiche Volumen an fibro-glandulärem Gewebe 7, 8 kleine Massen 9 auf unterschiedliche Art und Weise verdecken. In dem in FIG 1 dargestellten Beispiel ist das fibro-glanduläre Gewebe 7, das ein bestimmtes Volumen aufweist, an einer einzigen Stelle lokalisiert, so dass die kleine Masse 9 von dem dichten Gewebe 7 verdeckt wird. Aufgrund des Volumens des fibro-glandulären Gewebes 7 wird der dargestellte Bereich der Brust 3 durch einen bestimmten VBD-Wert charakterisiert. Währenddessen ist in dem in FIG 2 dargestellten Beispiel das fibro-glanduläre Gewebe 8, das ein identisches Volumen aufweist, gleichmäßiger in dem Volumen der Brust 3 verteilt, so dass es weniger wahrscheinlich ist, dass die kleine Masse 9 durch das dichte Gewebe 8 verdeckt wird. Trotzdem die VBD-Werte identisch sind, ist unmittelbar ersichtlich, dass das Risiko einer Maskierung der kleinen Masse 9 in FIG 2 geringer ist. Für eine genaue Beschreibung des Maskierungseffekts von mammographisch dichtem Gewebe 7, 8 ist daher die alleinige Verwendung des VBD-Wertes nicht ausreichend.

In der 5. Auflage des ACR BI-RADS-Atlas werden aus diesem Grund neue Kategorien "a" bis "d" mit einer verbalen Beschreibung der Brustdichtekategorie vorgeschlagen, definiert durch den visuell geschätzten Anteil an fibro-glandulärem Gewebe in der Brust. In diesem Zusammenhang wird erstmals vorgeschlagen, das Maskierungsrisiko zu berücksichtigen. So soll die Kategorie "c" vergeben werden, wenn aufgrund einer ungleichmäßigen Dichteverteilung kleine Massen verdeckt sein könnten. Es wird vorgeschlagen, daß der Radiologe in einem solchen Fall die Position des dichten Gewebes in einem weiteren Satz beschreibt.

Außerdem ist die Beschaffenheit der Brust mit der Kategorie "a" zu beschreiben, wenn die Brüste nahezu vollständig fetthaltig sind. Die Kategorie "b" soll vorliegen, wenn es verstreute Bereiche fibro-grandulärer Dichte gibt. Die Kategorie "d" soll vergeben werden, wenn die Brüste extrem dicht sind, wodurch die Empfindlichkeit der Mammographie verringert ist.

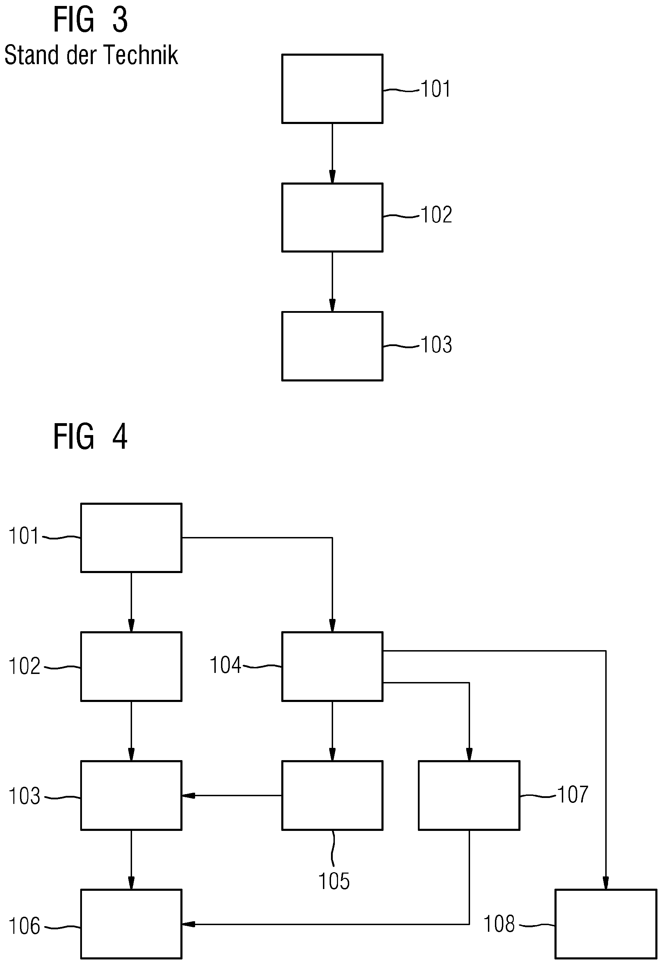

Wie in FIG 3 dargestellt, wird bisher unter Verwendung des während einer Mammographie erzeugten Röntgenbildes in einem ersten Schritt 101 eine Glandularitätskarte berechnet, wobei die Glandularität den Anteil des fibro-glandulären Gewebes an dem Gesamtgewebe bezeichnet. Die Glandularitätskarte definiert die Menge glandulären Gewebes über jedem Bildpixel des Röntgenbildes, entweder als Angabe in Millimeter oder als prozentuale Angabe, wobei der Wert typischerweise in einem Bereich von 1% bis 50% liegt. Dann wird in einem zweiten Schritt 102 die durchschnittliche Glandularität, der VBD-Wert, ermittelt. Anschließend erfolgt in einem dritten Schritt 103 die Bestimmung der Kategorien "a" bis "d" basierend allein auf dem VBD-Wert. Diese Schritte werden entweder manuell durch einen Radiologen oder bereits automatisiert mit Hilfe vorhandener Systeme durchgeführt. Nur erfahrene Radiologen können jedoch eine zuverlässige Beurteilung des Maskierungsrisikos vornehmen und einen vollständigen Brustdichtebericht unter Berücksichtigung des Maskierungsrisikos entsprechend den Vorgaben der ACR erstellen. Dabei besteht stets die Gefahr von Fehleinschätzungen.

Eine Aufgabe der vorliegenden Erfindung ist es daher, eine präzisere Auswertung eines während einer Mammographie erzeugten Röntgenbildes einer Brust hinsichtlich der Brustdichte zu ermöglichen. Diese Aufgabe wird durch ein Verfahren nach Anspruch 1 bzw. eine Vorrichtung nach Anspruch 8 bzw. ein Computerprogramm nach Anspruch 9 gelöst. Vorteilhafte Ausführungen der Erfindung sind in den Unteransprüchen angegeben. Die im Folgenden im Zusammenhang mit dem Verfahren erläuterten Vorteile und Ausgestaltungen gelten sinngemäß auch für die erfindungsgemäße Vorrichtung und umgekehrt.

Die Erfindung geht davon aus, die Maskierungseigenschaften des fibrograndulären Gewebes bei der Brustdichtebestimmung stärker zu berücksichtigen. Eine Kernidee der Erfindung ist es, die Bewertung des Röntgenbildes unter Berücksichtigung des Maskierungsrisikos und weitgehend, vorzugsweise vollständig automatisiert vorzunehmen. Zu diesem Zweck wird das von mammographisch dichtem Gewebe hervorgerufene Maskierungsrisiko automatisch bestimmt und zur Kategorisierung, Beschreibung und/oder Darstellung der Brustdichte verwendet. Mit der von der Erfindung vorgeschlagenen automatischen Quantifizierung des Maskierungsrisikos wird eine klinisch anwendbare, standardisierte Methode für eine einfache und verbesserte Beschreibung der Brustdichte bereitgestellt.

Erfindungsgemäß wird hierzu eine Maskierungsrisiko-Karte für den in dem Röntgenbild dargestellten Bereich der Brust automatisch erzeugt, wobei bei der Erzeugung der Maskierungsrisiko-Karte die Konnektivität dichten Gewebes durch Anwendung eines morphologischen Öffnungsverfahrens berücksichtigt wird. Auf dieser Grundlage wird ein Maskierungsrisiko-Wert (M-Wert) automatisch erzeugt, der das Maskierungsrisiko für den in dem Röntgenbild dargestellten Bereich der Brust quantifiziert. Vorteilhafterweise wird dieser M-Wert in Verbindung mit dem bereits bisher genutzten VBD-Wert verwendet, um eine Brustdichtekategorie gemäß BI-RADS, 5. Auflage, präziser zu bestimmen.

Die erzeugte Maskierungsrisiko-Karte wird in einer bevorzugten Ausführungsform der Erfindung gemeinsam mit dem Röntgenbild auf einem Bildschirm angezeigt, um diejenigen Bereiche des Bildes, für die ein erhöhtes Maskierungsrisiko ermittelt wurde, anzuzeigen. Ein Radiologe kann die Informationen zum Maskierungsrisiko nutzen, um sich während der Auswertung des Röntgenbildes auf diese Bereiche zu konzentrieren.

Basierend auf der Maskierungsrisiko-Karte wird in einer bevorzugten Ausführungsform der Erfindung eine Beschreibung der Position und/oder der Verteilung von mammographisch dichtem Gewebe für den in dem Röntgenbild dargestellten Bereich der Brust automatisch erzeugt, vorteilhafterweise in Form eines kurzen Satzes, wie er als Teil des Brustdichteberichtes gemäß BI-RADS, 5. Auflage, verwendet werden kann.

Besonders vorteilhaft ist es, wenn einige oder alle der zuvor genannten Anwendungen miteinander kombiniert werden.

Mit der vorliegenden Erfindung ist es erstmals möglich, einen quantifizierten, automatisch erstellten Brustdichtebericht unter Berücksichtigung des Maskierungsrisikos zu erzeugen, der den Anforderungen gemäß ACR BI-RADS, 5. Auflage, genügt. Die Genauigkeit der vorzunehmenden Klassifizierung in Brustdichtekategorien ist gegenüber herkömmlichen Lösungen erhöht. Subjektive Beeinflussungen und Fehler werden reduziert oder ausgeschlossen. Die Bewertung erfolgt auf konsistente und reproduzierbare Art und Weise. Insgesamt wird mit der Erfindung eine sehr viel einfachere Auswertung eines während einer Mammographie erzeugten Röntgenbildes einer Brust hinsichtlich der Brustdichte erreicht.

Die Erfindung ist besonders vorteilhaft anwendbar bei digitalen Mammographiegeräten sowie im Bereich bildgestützter, vorzugsweise automatisierter Brustdichtemessungen.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Dabei zeigen:

　　FIG 1 und 2 eine Darstellung des Maskierungseffekts kleiner Massen durch unterschiedlich angeordnetes fibro-glanduläres Gewebe,

　　FIG 3 den herkömmlichen Ablauf der Erstellung eines Brustdichteberichts (Stand der Technik),

　　FIG 4 den Ablauf der Erstellung eines Brustdichteberichts gemäß der Erfindung,

　　FIG 5 eine Glandularitätskarte einer Brust,

　　FIG 6 ein Bild einer Brust nach einer Schwellwertbehandlung,

　　FIG 7 ein Bild einer Brust nach einer morphologischen Öffnung,

　　FIG 8 die Darstellung einer Abbildungsfunktion f, wie sie zur Berechnung des M-Wertes verwendet wird,

　　FIG 9 eine Maskierungsrisiko-Karte,

　　FIG 10 eine Darstellung von VBD-Werten zu den Brustdichtekategorien,

　　FIG 11 eine erfindungsgemäße Vorrichtung.

Sämtliche Figuren zeigen die Erfindung lediglich schematisch und mit ihren wesentlichen Bestandteilen. Gleiche Bezugszeichen entsprechen dabei Elementen gleicher oder vergleichbarer Funktion.

Bei einem erfindungsgemäßen Verfahren zur Auswertung eines während einer Mammographie erzeugten Röntgenbildes 10 einer Brust 3 wird das von mammographisch dichtem Gewebe 7, 8 hervorgerufene Maskierungsrisiko automatisch bestimmt und zur Kategorisierung, Beschreibung und/oder Darstellung der Brustdichte verwendet. Bei dem auszuwertenden Röntgenbild 10 handelt es sich vorzugsweise um ein Vollfeldmammographie (FFDM)-Bild oder um ein Projektionsbild einer digitalen Brusttomosynthese (DBT)-Aufnahme.

Wird nachfolgend auf ein "Röntgenbild" Bezug genommen, muß es sich nicht immer um die gesamte Aufnahme handeln. Statt dessen kann es sich auch nur um einen aktuell angezeigten oder bearbeiteten Bildbereich des aufgenommenen Röntgenbildes handeln.

Grundlage des erfindungsgemäßen Verfahrens ist die automatische Erzeugung einer Maskierungsrisiko-Karte 11 für den in dem Röntgenbild dargestellten Bereich der Brust 3, siehe FIG 4, Schritt 104. In dieser Maskierungsrisiko-Karte 11 wird das miteinander verbundene, dichte Gewebe gezeigt. Nachfolgend wird ein beispielhafter Weg zur Erzeugung einer solchen Maskierungsrisiko-Karte 11 beschrieben.

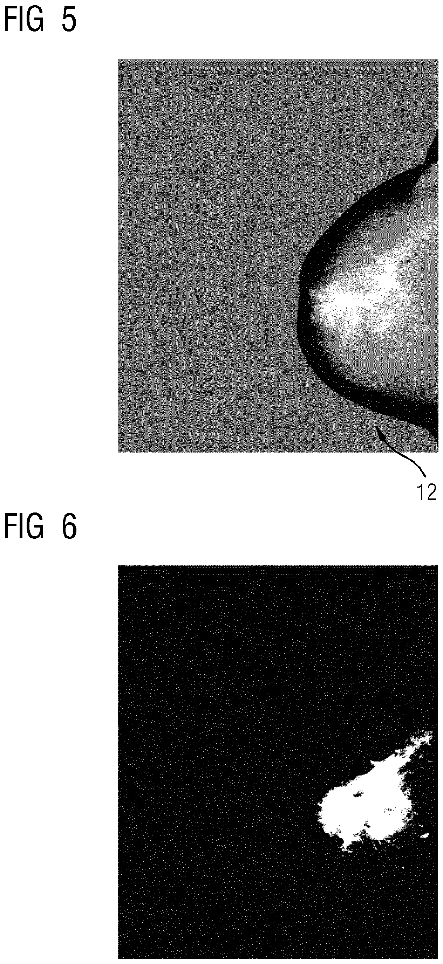

Als Eingangsdaten zur Ermittlung der Maskierungsrisiko-Karte 11 dienen die Daten einer Glandularitätskarte 12 ($G(x,y)$), wie sie beispielhaft in FIG 5 abgebildet ist. Bei der nachfolgenden Beschreibung wird davon ausgegangen, dass die Glandularität in Prozent angegeben ist. Eine Umrechnung der Glandularität in die Einheit mm ist durch Multiplikation des Prozentwertes mit der bekannten Dicke der komprimierten Brust, gemessen in mm, einfach durchzuführen. Es wird weiter davon ausgegangen, dass Strukturen, die für die Berechnung der Brustdichte nicht relevant sind, wie beispielsweise der Brustmuskel oder die Mamille, segmentiert und aus der Glandularitätskarte 12 entfernt wurden.

Es gibt verschiedene, dem Fachmann bekannte Möglichkeiten, eine solche Glandularitätskarte 12 zu berechnen. Die Art und Weise der Berechnung der Glandularitäten ist nicht Bestandteil dieser Erfindung. Statt dessen beschäftigt sich die vorliegende Erfindung mit der Auswertung der Glandularitätskarte 12, wie nachfolgend genauer beschrieben.

Bei der Berechnung der Maskierungsrisiko-Karte 11 wird die Glandularitätskarte 12 in den (in FIG 4 nicht einzeln dargestellten) Teilschritten 104a, 104b und 104c des Schrittes 104 auf unterschiedlichen Schwellwert-Ebenen mehrfach verarbeitet. Die Anzahl der Durchläufe hängt dabei von der Anzahl der gewünschten Schwellwert-Ebenen ab. Beispielsweise umfasst eine erste Schwellwert-Ebene Brustdichtewerte zwischen 10% und 15% und eine zweite Schwellwert-Ebene umfasst Brustdichtewerte zwischen 15% und 20% usw. Die Schwellwert-Ebenen können sich dabei überlappen. In dem nachfolgenden Beispiel umfasst die erste Schwellwert-Ebene Brustdichtewerte über 15%, die zweite Schwellwert-Ebene umfasst Brustdichtewerte über 20% und die dritte Schwellwert-Ebene umfasst Brustdichtewerte über 25% (N = 3). Die Teilschritte 104a, 104b und 104c werden N-mal ausgeführt (i = 1, ..., N), einmal für jede Schwellwert-Ebene.

In dem ersten Teilschritt 104a erfolgt eine Binärsegmentierung in einen Vordergrund- und einen Hintergrundbereich. Dabei wird das binäre Bild $I_i^{sg}(x,y)$ durch eine Schwellwertbehandlung der Glandularitätskarte 12 mit dem Schwellwert $T_i=\{15\%; 20\%; 25\%\}$ erzeugt. Dabei wird angenommen, dass die T-Werte in aufsteigender Reihenfolge sortiert sind, d. h. die Schwellwert-Ebenen Bereiche mit ansteigender Dichte repräsentieren. In dem erzeugten Bild $I_i^{sg}(x,y)$ sind damit alle Bildpixel markiert, in denen die Dichte größer ist als der gegebene Schwellwert $T_i$. An den Orten dieser Bildpixel besteht ein potentielles Risiko, dass kleine Massen 9 verdeckt werden. FIG 6 zeigt beispielhaft ein Bild $I_1^{sg}(x,y)$.

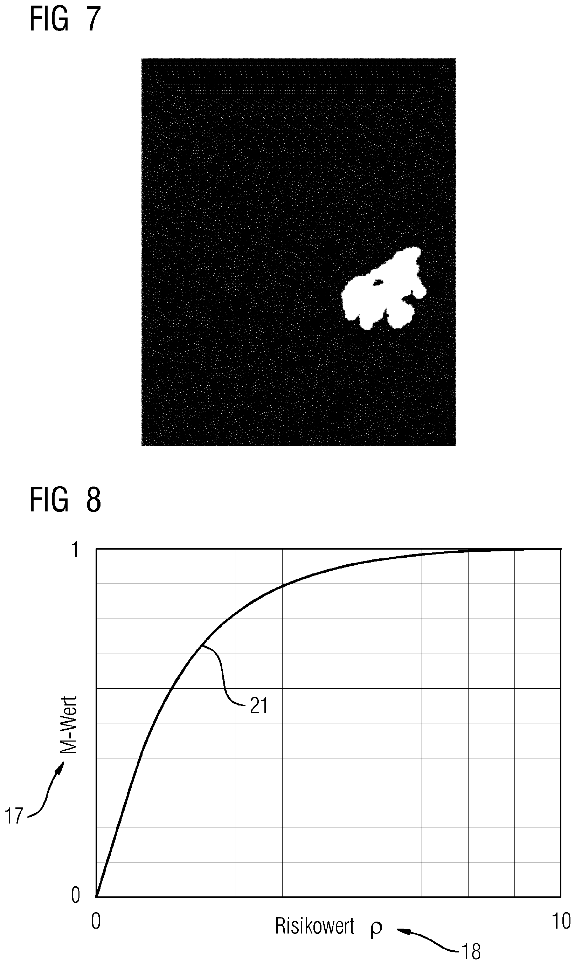

Bisher blieb jedoch die Konnektivität des dichten Gewebes unberücksichtigt. Diese ist hier von Bedeutung, da ein kleines isoliertes Gebiet dichten Gewebes mit einer Glandularität, die größer oder gleich dem Schwellwert $T_i$ ist, bei dem verwendeten Ansatz der Schwellwertbehandlung segmentiert würde, trotzdem es zu klein ist, um tatsächlich eine kleine Masse 9 zu verdecken. Die Konnektivität des dichten Gewebes wird daher bei der Erzeugung der Maskierungsrisiko-Karte 11 mit berücksichtigt. Dies erfolgt, indem in dem sich daran anschließenden Teilschritt 104b ein morphologisches Öffnungsverfahrens angewendet wird. Dabei wird das Bild $I_i^{op}(x,y)$ durch morphologisches Öffnen des Bildes $I_i^{sg}(x,y)$ mit einem scheibenförmigen Strukturelement mit dem Radius $R_i$ berechnet. Mit diesem Teilschritt werden vergleichsweise kleine isolierte Bereiche dichten Gewebes, bei denen es aufgrund ihrer Größe unwahrscheinlich ist, dass sie kleine Massen 9 verdecken, entfernt. Es wird dabei ein scheibenförmiges Strukturelement verwendet, da angenommen wird, dass die Massen 9 in erster Linie eine runde Form aufweisen. FIG 7 zeigt beispielhaft ein Bild $I_1^{op}(x,y)$.

Die Anwendung dieses Teilschrittes ist auch deshalb von besonderem Vorteil, weil hierdurch auch kleine, isolierte, konstrastreiche Objekte, wie beispielsweise Mikroverkalkungen oder Metallklammern, die in der Glandularitätskarte 12 enthalten sein können, entfernt werden.

Besonders vorteilhaft ist es, wenn während des morphologischen Öffnungsverfahrens dieselben Strukturelemente für das Abtragen wie auch für das erneute Hinzufügen verwendet werden, da dies als Standardverfahren bereits in Software-Bibliotheken implementiert ist. Im Beispiel beträgt der Radius $R_i=\{0,4\text{cm}; 0,4\text{cm}; 0,4\text{cm}\}$. Es ist ebenfalls möglich, scheibenförmige Strukturelemente mit unterschiedlichen Radien für das Entfernen und das Hinzufügen zu verwenden. Besonders vorteilhaft ist es, wenn der Radius für das Hinzufügen kleiner als der des Abtragens ist, da dann die Endfläche immer etwas kleiner als die Ursprungsfläche sein wird. Dadurch können Bereiche entfernt werden, in denen ein Teil der Masse in einem dichten Bereich, ein anderer Teil der Masse jedoch außerhalb des dichten Bereiches liegt, so daß lediglich das Risiko einer weniger kritischen Teilmaskierung besteht. Vorteilhafterweise wird in diesem Zusammenhang für das Hinzufügen ein Strukturelement verwendet, dessen Radius einem bestimmten Bruchteil des Radius desjenigen Strukturelements entspricht, das für das Abtragen verwendet wird. Als besonders vorteilhaft hat es sich dabei erwiesen, wenn der Radius des für das Hinzufügen verwendeten Strukturelements einen Wert von 70% bis 80% des Radius des für das Abtragen verwendeten Strukturelements entspricht.

In dem Teilschritt 104c wird die Fläche $A_i$ des verbleibenden Vordergrundbereiches in dem Bild $I_i^{op}(x,y)$ berechnet, also desjenigen Bereiches, der solche Bildpixel aufweist, in denen die Dichte größer als der jeweilige Schwellwert ist. Diese Fläche $A_i$ (in der Einheit $cm^2$) ist ein Maß für das Risiko, für die jeweilige Schwellwert-Ebene, dass das dichte Gewebe eine kleine Masse 9 verdeckt. Die in FIG 7 dargestellte Fläche ist beispielsweise $A_1$ = 13,5 cm². Die weiteren Flächen betragen beispielsweise $A_2$ = 6,3 cm² und $A_3$ = 3,1 cm².

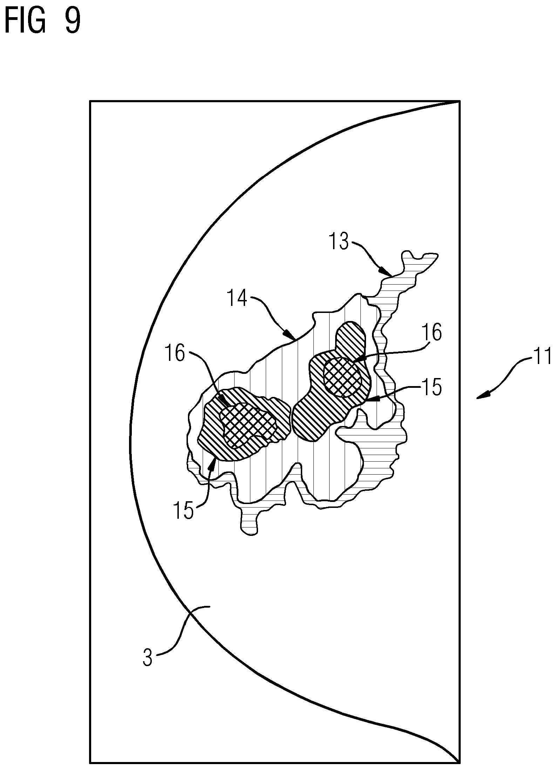

Aus den erhaltenen Bildern $I_i^{op}(x,y)$ mit i = 1, ..., N wird nach dem mehrmaligen Durchlaufen der Teilschritte 104a bis 104c in dem abschließenden (in FIG 4 ebenfalls nicht einzeln dargestellten) Teilschritt 104d des Schrittes 104 die zweidimensionale Maskierungsrisiko-Karte 11 erstellt, in der jedem Bildpixel (x,y) der größtmögliche Wert i aus dem Bildersatz $I_i^{op}(x,y)$ zugewiesen wird, bei dem dieser Bildpixel noch segmentiert, d. h. als Vordergrundbereich klassifiziert wurde. Ein Beispiel für eine derartige Maskierungsrisiko-Karte 11 ist in FIG 9 abgebildet. Auf der dunkelblau dargestellten Brust, deren Glandularitätskarte 12 in FIG 5 abgebildet ist, ist dichtes Brustgewebe 13, das wegen seiner begrenzten Konnektivität bei der Bewertung des Maskierungsrisikos unberücksichtigt geblieben war, hellblau abgebildet. Dichtes Gewebe 14 mit einer Glandularität über 15% ist gelb, dichtes Gewebe 15 mit einer Glandularität über 20% orange und dichtes Gewebe 16 mit einer Glandularität über 25% rot dargestellt. Andere Farbkodierungen können verwendet werden.

Anschließend wird für den in dem Röntgenbild 10 dargestellten Bereich der Brust ein Maskierungsrisiko-Wert (M-Wert) 17 automatisch erzeugt, siehe FIG 4, Schritt 105. Dieser M-Wert 17 quantifiziert das Maskierungsrisiko für diesen Bereich der Brust 3. Dabei bedeutet ein geringer M-Wert 17, dass die Wahrscheinlichkeit einer Maskierung einer Masse 9 gering ist, während ein hoher M-Wert 17 eine hohe Maskierungswahrscheinlichkeit bedeutet. Der Radiologe könnte im Fall eines hohen M-Wertes 17 beispielsweise die Durchführung einer weiterführenden Untersuchung empfehlen, beispielsweise einer Brust-Ultraschalluntersuchung oder einer Brust-Tomosynthese. Nachfolgend wird ein beispielhafter Weg zur Erzeugung eines solchen M-Wertes 17 beschrieben.

In einem ersten Teilschritt 105a werden die berechneten Flächen $A_i$ verwendet, um einen (dimensionslosen) Gesamt-Risikowert ($\rho$) 18 zu berechnen. Dies erfolgt beispielsweise durch Anwendung eines linearen Risikomodells

$$\rho = \frac{1}{N}\sum_{i=1}^{N} c_i A_i$$

wobei die Koeffizienten $c_i$ ansteigende (oder zumindest nicht fallende) Werte aufweisen, im Beispiel $c_i$={2cm⁻²; 5cm⁻²; 8cm⁻²}. Aufgrund dieser gewichteten Summation tragen die Flächen mit höherer Dichte stärker zum Gesamtrisiko bei. In dem hier beschriebenen Beispiel beträgt $\rho$ = 2,52. Anstelle eines linearen Risikomodells können auch andere, beispielsweise nichtlinearen Modelle verwendet werden.

Da die Interpretation des Gesamt-Risikowertes $\rho$ einfacher ist, wenn der Wert auf eine numerische Skala von 0% bis 100% abgebildet ist, erfolgt in dem sich anschließenden Teilschritt 105b eine Normierung unter Verwendung der Abbildungsfunktion (f) 21, wodurch der Maskierungsrisiko-Wert (M) 17 erzeugt wird. Dabei gilt M = f($\rho$).

Bei der Abbildungsfunktion 21 handelt es sich vorzugsweise um eine nichtlineare Funktion f($\rho$) = 1-exp(-$\alpha\rho$) mit $\alpha$ = 0,57, wie sie in FIG 8 dargestellt ist. Von Vorteil bei einer solchen nichtlinearen Funktion ist es, dass ihr Verhalten annährend stückweise linear ist. Dabei ist die Funktion stets steigend derart, dass ansteigende p-Werte immer zu größeren M-Werten führen, und die Funktion erreicht asymptotisch 100%. Es können aber auch andere Abbildungsfunktionen f verwendet werden. In dem hier beschriebenen Beispiel beträgt M = 74%.

Anschließend wird der M-Wert 17 verwendet, um dem in dem Röntgenbild 10 dargestellten Bereich der Brust eine Brustdichtekategorie 20 zuzuweisen, siehe FIG 4, Schritt 103. Die Zuweisung erfolgt vorzugsweise vollautomatisch. In einer anderen Ausführungsform der Erfindung erfolgt die Zuweisung halbautomatisch. Das bedeutet, dass dem Radiologen automatisch ein Vorschlag unterbreitet wird, dem der Radiologe zustimmen oder den der Radiologe abändern kann.

Die Zuweisung einer Brustdichtekategorie 20 zu dem Röntgenbild 10 erfolgt unter Verwendung und durch Verknüpfung des VBD-Wertes 19, der typischerweise in einem Bereich zwischen 2% und 30% liegt, und dem M-Wert 17, der einen Wert zwischen 0% und 100% annehmen kann. Da man davon ausgehen kann, dass niedrige VBD-Werte 19 zu niedrigen M-Werten 17 und hohe VBD-Werte 19 zu hohen M-Werten 17 führen, ist es sinnvoll, den M-Wert 17 bei der Beurteilung des mittleren Wertebereiches der VBD-Werte 19 einzusetzen. Dabei ist die Unterscheidung der beiden Brustdichtekategorien "b" und "c" von besonderer Bedeutung, da die beiden Kategorien "c" und "d" jeweils "dichte" Brüste betreffen und eine zusätzliche Untersuchung nach den existierenden Richtlinien empfehlenswert ist.

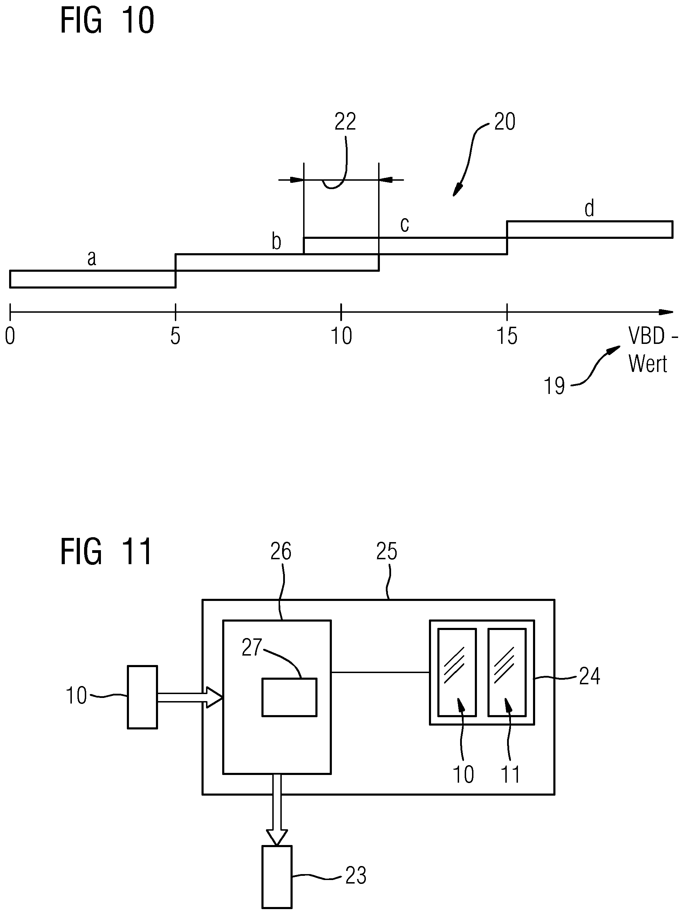

Es wird daher vorgeschlagen, dass der M-Wert 17 so verwendet wird, dass er ausschließlich die Zuordnung der Brustdichtekategorien "b" und "c" beeinflusst. In FIG 10 ist illustriert, in welchem Bereich 22 des VBD-Wertes 19 der M-Wert 17 auf die Klassifizierung in die einzelnen Brustdichtekategorien "a" bis "d" Einfluß nehmen soll. Dabei wird anstelle eines scharfen VBD-Schwellwertes von beispielsweise 10% zur Unterscheidung der benachbarter Kategorien "b" und "c", wie er bisher üblich war, ein flexibler Schwellwert zur Abgrenzung dieser beiden Brustdichtekategorien 20 vorgeschlagen. Dieser flexible Schwellwert kann sich beispielsweise über einen Bereich 22 von VBD-Werten 19 von 9,2% bis 10,8% erstrecken.

Die nachfolgende Tabelle verdeutlicht beispielhaft die Zuordnung einer bestimmten Brustdichtekategorie 20 in Abhängigkeit von dem jeweiligen M-Wert 17 bei nahezu identischen VBD-Werten 19 in diesem Schwellwertbereich 22.

| VBD-Wert | M-Wert | Brustdichtekategorie |
|----------|--------|----------------------|
| 9,2% | 88% | c |
| 9,4% | 23% | b |
| 10,2% | 23% | b |
| 10,7% | 54% | c |

Die Verwendung anderer Entscheidungsregeln ist möglich. Auch können andere Faktoren, wie beispielsweise das Alter der Frau, in die Klassifizierung einfließen.

Gleichzeitig mit der Berechnung des M-Wertes 17 wird für den in dem Röntgenbild 10 dargestellten Bereich der Brust 3 eine auf der Maskierungsrisiko-Karte 11 beruhende, d.h. unter Verwendung der Karte erstellte, Beschreibung der Position und/oder der räumlichen Verteilung von mammographisch dichtem Gewebe automatisch erzeugt, siehe FIG 4, Schritt 107. Dies betrifft insbesondere die räumliche Anordnung derjenigen Bereiche 14, 15, 16, deren Maskierungsrisiko sehr hoch ist, siehe die in FIG 9 rot dargestellten Bereiche 16. Mit Hilfe eines geeigneten Algorithmus wird die Maskierungsrisiko-Karte 11 automatisch analysiert und eine entsprechende Beschreibung in Textform, beispielsweise in Form eines kurzen Satzes, generiert und dem Brustdichtebericht 23 hinzugefügt. Von Vorteil ist dabei eine standardisierte Form einer verbalen Beschreibung, beispielsweise unter Verwendung einer Einteilung des Bildes in Quadranten.

Zusammen mit den in Schritt 103 bestimmten Brustdichtekategorien 20 und den in Schritt 107 erstellen Positionsbeschreibungen wird in dem abschließenden Schritt 106 ein korrekter Brustdichtebericht 23 entsprechend den Vorgaben der ACR erstellt.

Parallel dazu wird dem Radiologen die Maskierungsrisiko-Karte 11, siehe FIG 9, gemeinsam mit dem Röntgenbild 10 auf einem Bildschirm 24 angezeigt, siehe FIG 4, Schritt 108. Diese Informationen kann der Radiologe verwenden, um beispielsweise in Schritt 103 den automatisch erzeugten Vorschlag einer Brustdichtekategorie 20 besser bewerten zu können.

Dabei kann die Maskierungsrisiko-Karte 11 neben dem (verarbeiteten) FFDM-Bild oder dem rekonstruierten DBT-Datensatz angezeigt werden oder die Maskierungsrisiko-Karte 11 kann mit dem FFDM-Bild oder dem DBT-Datensatz verschmolzen werden. Beispielsweise kann das FFDM-Bild oder der DBT-Datensatz in den HSV-Farbraum konvertiert werden. In diesem Farbraum sind Grauwertinformationen in dem "V"-Kanal gespeichert. Die beiden anderen Kanäle "H" und "S" dienen zur Kodierung der Maskierungsrisiko-Karte 11. Durch Änderung der Werte in diesen beiden Kanälen ist es auf einfache Weise möglich, die Anzeigestärke der Maskierungsrisiko-Karte 11 als Overlay über dem FFDM-Bild bzw. dem DBT-Datensatz zu steuern. Auf diese Art und Weise kann der Radiologe die Maskierungsrisiko-Karte 11 besonders einfach in das Röntgenbild 10 ein- bzw. ausblenden. Ein Wechsel zwischen den beiden Ansichten im Fall einer Überlagerung der Bilder bzw. Karten kann aber auch auf andere Art und Weise bewerkstelligt werden.

Die in dem beschriebenen Ausführungsbeispiel beschriebene Werte der Parameter ($N$, $T_i$, $R_i$, $c_i$) können von anderen Parametern abhängig gemacht werden, beispielsweise von der komprimierten Brustdicke oder von Bestrahlungsparametern.

Handelt es sich bei dem Röntgenbild um eine DBT-Aufnahme, dann liegen typischerweise n = 25 aufgenommene Bilder vor, wobei vorzugsweise jedes Projektionsbild einzeln ausgewertet wird. Ebenso vorzugsweise werden die aus allen n Projektionen erhaltenen Informationen verwendet, um eine gemeinsame Maskierungsrisiko-Karte 11, einen gemeinsamen VBD-Wert 21 und einen gemeinsamen M-Wert 17 zu berechnen. Dabei kann beispielsweise eine (gewichtete) Mittelung mit Beseitigung der Ausreißer verwendet werden.

Die erfindungsgemäße Vorrichtung 25 ist ausgebildet zur Durchführung des beschriebenen Verfahren und weist alle hierfür erforderlichen Mittel auf. Vorzugsweise umfaßt die Vorrichtung eine Datenverarbeitungseinheit 26, ausgebildet zur Durchführung aller Schritte entsprechend des hier beschriebenen Verfahrens, die in einem Zusammenhang mit der Verarbeitung von Daten stehen. Die Verarbeitungseinheit 26 weist vorzugsweise eine Anzahl von Funktionsmodulen auf, wobei jedes Funktionsmodul ausgebildet ist zur Durchführung einer bestimmten Funktion oder einer Anzahl bestimmter Funktionen gemäß dem beschriebenen Verfahren. Beispielsweise weist die Vorrichtung 25 einen Bildschirm 24 zur Anzeige des Röntgenbildes 10 und/oder der Maskierungsrisiko-Karte 11 auf. Geeignete Ein- und Ausgabeeinrichtungen sind ebenso vorgesehen, wie Schnittstellen zur Eingabe des Röntgenbildes 10 und zur Ausgabe des Brustdichteberichtes 23.

Bei den Funktionsmodulen kann es sich um Hardwaremodule oder Softwaremodule handeln. Mit anderen Worten kann die Erfindung, soweit es die Verarbeitungseinheit 26 betrifft, entweder in Form von Computerhardware oder in Form von Computersoftware oder in einer Kombination aus Hardware und Software verwirklicht werden. Soweit die Erfindung in Form von Software, also als Computerprogramm 27, verwirklicht ist, werden sämtliche beschriebenen Funktionen durch Computerprogrammanweisungen realisiert, wenn das Computerprogramm 27 auf einem Rechner 26 mit einem Prozessor ausgeführt wird. Die Computerprogrammanweisungen sind dabei auf an sich bekannte Art und Weise in einer beliebigen Programmiersprache verwirklicht und können dem Rechner in beliebiger Form bereitgestellt werden, beispielsweise in Form von Datenpaketen, die über ein Rechnernetz übertragen werden, oder in Form eines auf einer Diskette, einer CD-ROM oder einem anderen Datenträger gespeicherten Computerprogrammprodukts.

Obwohl die Erfindung im Detail durch das bevorzugte Ausführungsbeispiel näher illustriert und beschrieben wurde, so ist die Erfindung nicht auf die offenbarten Beispiele eingeschränkt und andere Variationen können vom Fachmann hieraus abgeleitet werden, ohne den Schutzumfang der Erfindung zu verlassen.

Bezugszeichenliste

| | |
|---|---|
| 1 | Platte |
| 2 | Platte |
| 3 | Brust |
| 4 | Röntgenquelle |
| 5 | Röntgenstrahlung |
| 6 | Röntgendetektor |
| 7 | dichtes Gewebe |
| 8 | dichtes Gewebe |
| 9 | kleine Masse |
| 10 | Röntgenbild |
| 11 | Maskierungsrisiko-Karte |
| 12 | Glandularitätskarte |
| 13 | dichtes Gewebe (unberücksichtigt) |
| 14 | dichtes Gewebe (<15%) |
| 15 | dichtes Gewebe (<20%) |
| 16 | dichtes Gewebe (<25%) |
| 17 | M-Wert |
| 18 | Risikowert |
| 19 | VBD-Wert |
| 20 | Brustdichtekategorie |
| 21 | Abbildungsfunktion |
| 22 | VBD-Wertebereich |
| 23 | Brustdichtebericht |
| 24 | Bildschirm |
| 25 | Vorrichtung |
| 26 | Datenverarbeitungseinheit |
| 27 | Computerprogramm |

**Patentansprüche**

1. Verfahren zur Auswertung eines während einer Mammographie erzeugten Röntgenbildes (10) einer Brust (3), wobei das von mammographisch dichtem Gewebe (7, 8) hervorgerufene Maskierungsrisiko automatisch bestimmt und zur Kategorisierung, Beschreibung und/oder Darstellung der Brustdichte verwendet wird, indem eine Maskierungsrisiko-Karte (11) automatisch erzeugt wird, wobei die Maskierungsrisiko-Karte (11) einen Maskierungsrisiko-Wert (17) umfasst, der das Maskierungsrisiko quantifiziert, **dadurch gekennzeichnet, dass** bei der Erzeugung der Maskierungsrisiko-Karte (11) die Konnektivität dichten Gewebes (7, 8) durch Anwendung eines morphologischen Öffnungsverfahrens berücksichtigt wird.

2. Verfahren nach Anspruch 1, **dadurch gekennzeichnet, dass** zur Erzeugung der Maskierungsrisiko-Karte (11) die Daten einer Glandularitätskarte (12) verwendet werden.

3. Verfahren nach Anspruch 1 oder 2, **dadurch gekennzeichnet, dass** das im Rahmen des morphologischen Öffnungsverfahrens verwendete Strukturelement scheibenförmig ist und sich die Radien der für das Abtragen und das Hinzufügen verwendeten Strukturelemente voneinander unterscheiden.

4. Verfahren nach einem der Ansprüche 1 bis 3, **dadurch gekennzeichnet, dass** die Maskierungsrisiko-Karte (11) gemeinsam mit dem Röntgenbild (10) auf einem Bildschirm (24) angezeigt wird.

5. Verfahren nach einem der Ansprüche 1 bis 4, **dadurch gekennzeichnet, dass** ein auf der Maskierungsrisiko-Karte (11) beruhender Maskierungsrisiko-Wert (17) automatisch erzeugt wird.

6. Verfahren nach Anspruch 5, **dadurch gekennzeichnet, dass** der Maskierungsrisiko-Wert (17) verwendet wird, um dem in dem Röntgenbild (10) dargestellten Bereich der Brust (3) eine Brustdichtekategorie (20) zuzuweisen.

7. Verfahren nach einem der Ansprüche 1 bis 6, **dadurch gekennzeichnet, dass** eine auf der Maskierungsrisiko-Karte (11) beruhende Beschreibung der Position und/oder der Verteilung von mammographisch dichtem Gewebe (7, 8) automatisch erzeugt wird.

8. Vorrichtung (25) zur Auswertung eines während einer Mammographie erzeugten Röntgenbildes (10) einer Brust (3), **gekennzeichnet durch** Mittel (26, 24) zur Durchführung des Verfahrens nach einem der Ansprüche 1 bis 7, wobei diese Mittel eine Datenverarbeitungseinheit (26) sowie Schnittstellen zur Eingabe des Röntgenbildes (10) und zur Anzeige des Röntgenbildes (10) und/oder der Maskierungsrisiko-Karte (11) auf einem Bildschirm (24) umfassen.

9. Computerprogramm (27) zur Auswertung eines während einer Mammographie erzeugten Röntgenbildes (10) einer Brust (3), **gekennzeichnet durch** Computerprogrammanweisungen zur Durchführung des Verfahrens nach einem der Ansprüche 1 bis 7, wenn das Computerprogramm (27) auf einem Rechner (26) ausgeführt wird.

**Claims**

1. Method for evaluating an x-ray image (10) of a breast (3) produced during mammography, wherein the masking risk caused by mammographically dense tissue (7, 8) is determined automatically and used for categorizing, describing and/or representing the breast density, by virtue of a masking risk map (11) being produced automatically, wherein the masking risk map (11) comprises a masking risk value (17) that quantifies the masking risk, **characterized in that** the connectivity of dense tissue (7, 8) is taken into account when producing the masking risk map (11) by applying a morphological opening method.

2. Method according to Claim 1, **characterized in that** the data from a glandularity map (12) are used for producing the masking risk map (11).

3. Method according to Claim 1 or 2, **characterized in that** the structure element used within the scope of the morphological opening method is disc-shaped and the radii of the structure elements used for ablation and addition differ from one another.

4. Method according to one of Claims 1 to 3, **characterized in that** the masking risk map (11) is displayed on a screen (24) together with the x-ray image (10).

5. Method according to one of Claims 1 to 4, **characterized in that** a masking risk value (17) based on the masking risk map (11) is produced automatically.

6. Method according to Claim 5, **characterized in that** the masking risk value (17) is used to assign a breast density category (20) to the region of the breast (3) depicted in the x-ray image (10).

7. Method according to one of Claims 1 to 6, **characterized in that** a description of the position and/or the distribution of mammographically dense tissue (7, 8) based on the masking risk map (11) is produced automatically.

8. Apparatus (25) for evaluating an x-ray image (10) of a breast (3) produced during mammography, **characterized by** means (26, 24) for carrying out the method according to one of Claims 1 to 7, wherein these means comprise a data processing unit (26) and interfaces for inputting the x-ray image (10) and for displaying the x-ray image (10) and/or the masking risk map (11) on a screen (24).

9. Computer program (27) for evaluating an x-ray image (10) of a breast (3) produced during mammography, **characterized by** computer program instructions for carrying out the method according to one of Claims 1 to 7 when the computer program (27) is executed on a computer (26).

**Revendications**

1. Procédé d'évaluation d'une radiographie (10) d'un sein (3) produite pendant une mammographie, dans lequel on détermine automatiquement le risque de masquage provoqué par du tissu (7, 8) dense mammographiquement et on l'utilise pour la catégorisation, la description et/ou la représentation de la densité du sein par le fait que l'on produit automatiquement une carte (11) de risque de masquage, la carte (11) de risque de masquage comprenant une valeur (17) de risque de masquage, qui quantifie le risque de masquage, **caractérisé en ce que**, lors de la production de la carte (11) de risque de masquage, on tient compte de la connectivité du tissu (7, 8) dense par application d'un procédé d'ouverture morphologique.

2. Procédé suivant la revendication 1, **caractérisé en ce que** pour la production de la carte (11) de risque de masquage, on utilise les données d'une carte (12) de glandularité.

3. Procédé suivant la revendication 1 ou 2, **caractérisé en ce que** l'élément de structure, utilisé dans le cadre du procédé d'ouverture morphologique, est en forme de disque et les rayons des éléments de structure, utilisés pour la soustraction et l'addition, sont différents les uns des autres.

4. Procédé suivant l'une des revendications 1 à 3, **caractérisé en ce qu'**on affiche la carte (11) de risque de masquage conjointement avec la radiographie (10) sur un écran (24).

5. Procédé suivant l'une des revendications 1 à 4, **caractérisé en ce que** l'on produit automatiquement une valeur (17) de risque de masquage reposant sur la carte (11) de risque de masquage.

6. Procédé suivant la revendication 5, **caractérisé en ce que** l'on utilise la valeur (17) de risque de masquage pour attribuer une catégorie (20) de densité de sein à la partie du sein (3) représentée dans la radiographie (10).

7. Procédé suivant l'une des revendications 1 à 6, **caractérisé en ce que** l'on produit automatiquement une description, reposant sur la carte (11) de risque de masquage, de la position et/ou de la répartition du tissu (7, 8) dense mammographiquement.

8. Installation (25) d'évaluation d'une radiographie (10) d'un sein (3) produite pendant une mammographie, **caractérisée par** des moyens (26, 24) pour effectuer le procédé suivant l'une des revendications 1 à 7, dans laquelle ces moyens comprennent une unité (26) de traitement de données, ainsi que des interfaces d'entrée de la radiographie (10) et d'affichage de la radiographie (10) et/ou de la carte (11) de risque de masquage sur un écran (24).

9. Programme (27) d'ordinateur pour l'évaluation d'une radiographie (10) d'un sein (3) produite pendant une mammographie, **caractérisé par** des instructions de programme d'ordinateur pour effectuer le procédé suivant l'une des revendications 1 à 7, lorsque le programme (27) d'ordinateur est réalisé sur un ordinateur (26).

FIG 1

FIG 2

## FIG 3
Stand der Technik

101

102

103

## FIG 4

101

102          104

103          105          107

106                        108

FIG 5

12

FIG 6

FIG 7

FIG 8

FIG 9

FIG 10

FIG 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110026791 A1
- DE 102006021042 A1